# EUROPEAN PATENT APPLICATION

(11) **EP 1 867 358 A1**
(43) Date of publication of application: **19.12.2007**
(21) Application number: 06012085.4
(22) Date of filing: 13.06.2006
(51) Int. Cl.: A61M 25/10, A61F 2/06

(54) **Instrument for expanding a stent**

(71) Applicant: DSM IP Assets B.V., 6411 TE Heerlen (NL)
(72) Inventor: The designation of the inventor has not yet been filed
(74) Representative: Habets, Winand

(57) **Abstract**

Instrument for expanding a stent (10), which has been inserted in a channel like organ, such as a blood vessel. The instrument comprises an inflatable balloon (1), provided with at least one flow passage forming region that, upon inflation of the balloon, provides at least one flow passage through the balloon-stent combination. The flow passage forming region may comprise a series of ribs (5), extending substantially radially, at regularly spaced intervals around the circumference of the balloon. Preferably, the ribs are twisted along part of the balloon's circumference.
The application furthermore relates to a kit of parts, including such an instrument and a stent.

## Description

The invention relates to an instrument for expanding a stent and/or a channel like organ, such as a blood vessel, by means of an inflatable balloon.

Such instruments are known. With such instrument, a balloon may be inserted in a blood vessel or other channel and manoeuvred to a desired location in a collapsed condition. Thanks to such collapsed condition, the channel will barely be obstructed and may function more or less as normal. Upon arrival at the desired location, the balloon is inflated, so as to unblock the channel, or expand a surrounding stent, or any other device that may be delivered by a balloon catheter. During such inflation, the balloon will substantially block the passage way through the channel and as such interrupt any flow there through. Since such flow is usually of vital importance, such interruption may only be very brief. This puts great pressure on the operating surgeon and moreover requires the balloon to be inflated quickly, with high inflating pressures. The impact caused by such inflating pressures may cause trauma to the surrounding channel wall. Moreover, if such instrument is used to implant a stent, such stent must be lockable in its expanded condition quickly. This limits the range of suitable stents. For instance, stents whose locking action is realized through curing, e.g. by (UV) light, (IR) radiation, heat or the like, will generally be too slow. The same applies for stents that make use of shape memory polymers, which may take tens of minutes or more to expand to their full diameter at body temperatures.

It is therefore an objective of the present invention to provide an instrument of the above mentioned type, with which the disadvantages of the known instruments are avoided or at least partly reduced.

To that end, an instrument according to the invention is characterized in that the balloon comprises at least one flow passage forming region that, during use, upon inflation of the balloon, provides at least one flow passage through the balloon and the surrounding stent and/or channel.

Thanks to such flow passage forming region, one or more flow passages may be formed upon inflation of the balloon, allowing the normal flow through the channel to be at least partly continued. As such, the inflation time of the balloon, i.e. the time during which the balloon is inflated, may be considerably increased. Consequently, more time is available to perform those operations for the benefit of which the balloon is inserted, for instance push aside plaque, delivery of a stent or any other device, anchoring, positioning, heat transfer, photodynamic therapy, balloon assisted drug delivery, etc. The increased inflation time furthermore allows for lower inflating pressures to be used, which will have a smaller impact on the surrounding channel. Also, more time is available to lock a stent in its expanded condition. This not only relieves the surgeon of possible operation stress, but also allows the application of a wider range of stents, featuring longer locking times, such as for instance stents whose locking action is based on curing or solidifying an initially soft or liquid locking medium, or stents made from slow expanding shape memory alloy materials.

The flow passage forming region may extend within the balloon, in which case the inflated balloon can for instance be of tubular shape. Alternatively or additionally, such flow passage forming region may be provided at the outer circumference of the balloon, for instance in the form of inflatable ribs, disposed at regular spaced intervals around said circumference. Upon inflation, the channel and/or stent will be expanded by said ribs, while flow passages are formed by the troughs between the ribs and the surrounding stent and/or channel wall.

According to a preferred aspect of the invention, the ribs may be twisted around part of the balloon's circumference. Since the expansion force exerted by the balloon on the channel wall or stent will be concentrated along the contact lines with the ribs, having the ribs twisted will help to distribute said expansion force more evenly along the circumference of the channel wall or stent. Thus, the channel and/or stent can be expanded substantially uniformly in radial direction, which in case of the stent may result in the expanded stent featuring a substantially circular cross section rather than a polygonal cross section. This in turn may enable the stent to exert a substantially homogenous, i.e. evenly distributed support pressure on the surrounding channel, thereby preventing local pressure peaks.

According to another preferred aspect the balloon may be made of a polymer material, wherein the at least one flow passage forming region may be reinforced by fibres. Generically, such fiber reinforcement may contribute toincreased balloon strength, leading to thinner walls, higher burst pressure and lower profile. By geometrically controlling the fiber composite direction and structure the local expansion and shape can be controlled, thereby allowing a variety of balloon shapes to be configured. Suitable fiber materials are for instance polymeric fibers from e.g. polyester (PET), Nylon (PA) or polypropylene (PP), or high performance fibers such as Kevlar, Dyneema (UHMw-PE), etc.

Suitable balloon materials are for instance polymers such as PET, Nylon, Polyurethane, thermoplastic elastomers from Nylon, Polyetheramide, Polyetherester, etc.

The invention furthermore relates to a kit of parts, including an instrument according to the invention and a stent. Said stent may be of any type. However, advantageously, the stent is of a type that features a relatively long locking time, such as for instance stents with locking means based on a curable material or stents that make use of shape memory alloy materials. The term 'locking time', in this description, refers to the time needed for locking means of the stent to take effect and allow the balloon to be removed, without the stent returning to its collapsed condition. With an instrument according to the invention, such stent can be slowly expanded, thereby reducing the risk of injuring the surrounding channel. Furthermore, the balloon may be kept in place as long as necessary for the locking means to take effect, without having to worry about interrupting vital flows through the channel.

Further advantageous embodiments of an instrument and a kit of parts according to the present invention are set forth in the dependent claims.

To explain the invention, exemplary embodiments thereof will hereinafter be described with reference to the accompanying drawings, wherein:
FIG. 1 shows in perspective view a balloon according to the invention, in inflated condition;
FIG. 2 shows the balloon of Figure 1, in operation, i.e. inserted within a stent;
FIG. 3 shows in perspective view, an alternative embodiment of a balloon according to the invention; and
FIG. 4 shows the balloon of Figure 3, in cross section, with reinforcing central ribs.

In the following description, embodiments of a balloon according to the invention will be described in combination with a stent. However, it is to be understood that the balloon may advantageously be used in many other medical applications and/or instruments, such as angioplasty procedures, occlusion, anchoring, positioning, PTCA catheters, PTA catheters, heat transfer catheters, photodynamic therapy, cryogenic catheters, drug delivery

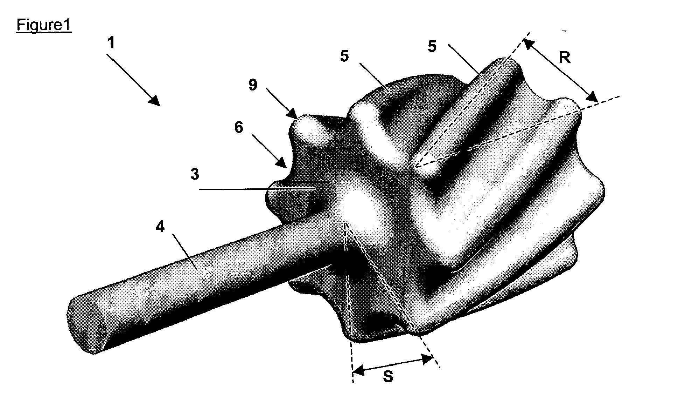

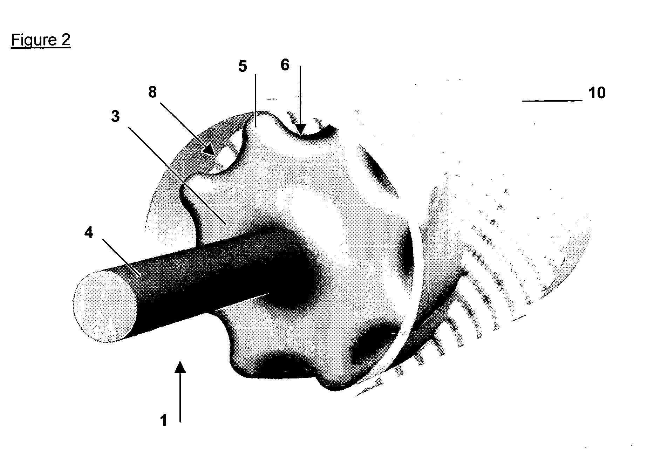

Figures 1 and 2 show a first embodiment of a balloon 1 according to the invention, suitable for expanding a stent 10. The balloon 1 comprises a substantially cylindrical main body 3, which at one end is connected to a tube 4 for pumping air (or some other pressurized medium) in and out off the balloon 1. The transition between the main body 3 and the tube 4 can be of any shape, e.g. conical, square, spherical, tapered or a combination thereof.

The balloon 1 furthermore comprises a series of ribs 5, which are arranged at regularly spaced intervals S around the circumference of the main body 3, and between which passage forming regions 6 are formed. In the illustrated embodiment, the balloon 1 is provided with eight ribs 5, but of course, in other embodiments this number can be varied.

In a preferred embodiment, as shown in Figure 1, the ribs 5 are slightly twisted around the circumference of the cylindrical main body 3, over a radial distance R that preferably at least equals aforementioned interval S between adjacent ribs 5. Furthermore, the ribs preferable have a somewhat rounded or flattened edge 9, so as to provide for a good contact area with the inner wall of stent 10.

During use, the balloon 1 will initially be deflated, whereby a stent may be mounted on the balloon 1, in collapsed condition. (This balloon-stent combination is inserted in a blood vessel or similar channel, and manoeuvred to a desired location. Upon reaching this location, the balloon 1 will be inflated via tube 4, causing the ribs 5 to expand the stent 10 to a substantially cylindrical shape (as shown in Figure 2), which can locally support the surrounding channel (not shown). Thanks to the twisted orientation of the ribs 5, the expansion force exerted on the stent 10 will be distributed substantially evenly over the inner circumference of the stent 10, resulting in the stent 10 being expanded substantially uniform in radial direction.

In the expanded condition of Figure 2, the ribs 5 prevent the stent 10 from returning to its collapsed condition, while the passage forming regions 6 form passages 8, via which vital flow circulation through the channel can be continued. Thanks to this passages 8 there is ample time to lock the stent 10 in its expanded condition, for instance by subjecting the stent 10 to a curing treatment or by allowing the stent to undergo some chemical or physical reaction (e.g. in case of shape memory materials that automatically expand at a certain temperature).

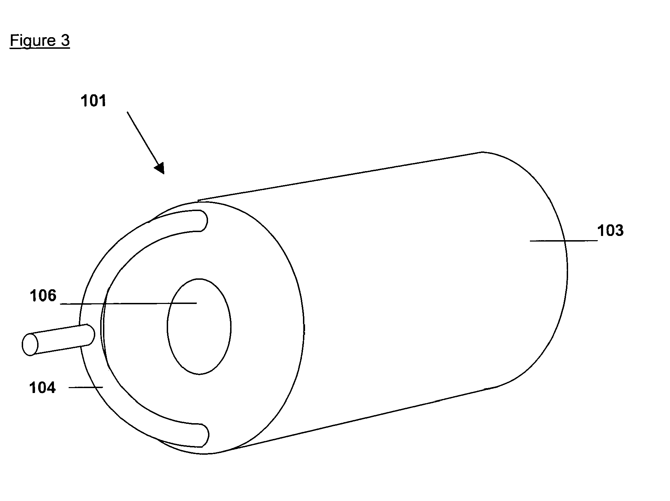

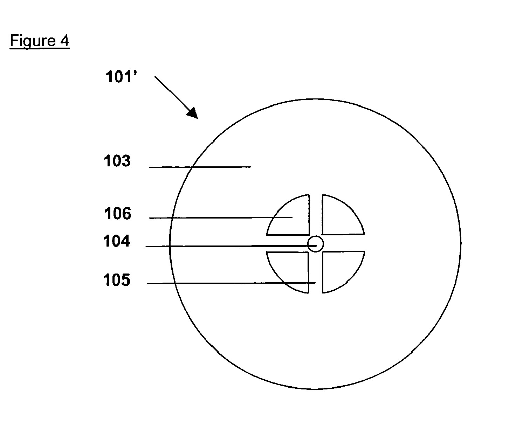

Figure 3 shows an alternative embodiment of a balloon 101 according to the invention. Like in the previous embodiment, the balloon 101 comprises a substantially cylindrical main body 103, which at one end is connected to a tube 104 for inflating and deflating the balloon 101. In this embodiment, the passage forming region extends completely within the balloon 101, in the form of a central opening 106 through the main body 103, thereby transforming the main body 103 in a hollow tube. To increase the radial expansion strength of the balloon 101, the central opening 106 may be provide with inflatable radial webs 105 as shown in Figure 4 (which shows a balloon 101' in cross section).

The balloon 101 functions similar to the embodiment of Figures 1 and 2. Upon inflation, the outer wall of the tubular main body 103 will abut and expand the stent 10, while the central opening 106 ensures any flow through the channel to be continued. As seen in Figure 3, the tube 104 for inflating and deflating the balloon 101 can be connected to several locations of the main body 103, so as to allow a substantially uniform inflation in radial direction. Of course, alternative embodiments are possible, having more or less connecting points than the one shown in Figure 3. In the embodiment of Figure 4, the tube may engage the balloon 101' centrally, at the junction of the webs 105. In all embodiments, the balloon 1, 101 can be fabricated of a polymer material, which at least near the passage forming regions 6, 106 may be reinforced with fibres. Thanks to such fibres, the passage forming regions 6, 106 can maintain a desired shape, even in inflated condition, when said regions experience substantial inflation pressure. In addition to the balloon 1, 101, as described above, the instrument according to the invention may comprise guiding means, for guiding the stent 10 to its desired location and/or activating means for activating any locking means of the stent. For instance, when the locking means of the stent need to be activated by a curing treatment, the instrument may be provided with heating means, radiation means or any other appropriate curing means. Of course, when the instrument is used for another medical application, as mentioned above, the instrument may be provided with all necessary means to perform said application, for example light treatments (e.g. laser balloon angioplasty), or drug delivery.
The invention is not in any way limited to the exemplary embodiments presented in the description and drawing. All combinations (of parts) of the embodiments shown and described are explicitly understood to be incorporated within this description and are explicitly understood to fall within the scope of the invention. Moreover, many variations are possible within the scope of the invention, as outlined by the claims.

## Claims

**1.** Instrument for treating a channel like organ, such as a blood vessel, said instrument comprising a balloon which during said treatment is inflated so as to have its outer circumference substantially match the inner circumference of the channel like organ, **characterised in that** the balloon comprises at least one flow passage forming region that, upon inflation of the balloon, provides at least one flow passage through the balloon and the surrounding channel like organ.

**2.** Instrument according to claim 1, wherein the at least one passage forming region extends within the balloon.

**3.** Instrument according to claim 1 or 2, wherein the at least one passage forming region extends at an outer circumference of the balloon.

**4.** Instrument according to anyone of the preceding claims, wherein the passage forming region comprises a series of ribs, extending substantially radially, at regularly spaced intervals around the circumference of the balloon.

**5.** Instrument according to claim 4, wherein the ribs are twisted along at least part of the balloon's circumference.

**6.** Instrument according to anyone of the preceding claims, wherein the passage forming region is made of fibre reinforced polymer material. 7. Kit of parts including an instrument according to anyone of the preceding claims and an expandable stent.

**8.** Kit of parts according to claim 7, wherein the stent comprises locking means with a relatively long locking time, such as for instance locking means that are based on curing some curable substance.

**9.** Use of an instrument according to anyone of claims 1-6, for anyone of the following treatments: delivery of a medical device for instance a stent, anchoring, positioning, heat transfer, photodynamic therapy, drug delivery, plaque control, or as part of a catheter, such as a PTCA catheter, a PTA catheter, a heat transfer catheter or a cryogenic catheter.
